# EUROPEAN PATENT APPLICATION

(11) **EP 4 652 919 A1**
(43) Date of publication of application: **26.11.2025**
(21) Application number: 23917838.7
(22) Date of filing: 10.07.2023
(51) Int. Cl.: A61B 5/00, G16H 50/20, G16H 30/40

(54) **METHOD AND SYSTEM FOR DIAGNOSING ORAL DISEASE OF COMPANION ANIMAL**

(30) Priority: 19.01.2023 KR 20230007839
(71) Applicant: AiForPet Co., Ltd., Pohang-si, Gyeongsangbuk-do 37666 (KR)
(72) Inventor: HEO, Euna, Seoul 06663 (KR)
(74) Representative: AWA Sweden AB
(86) International application number: PCT/KR2023/009775
(87) International publication number: WO 2024/154880

(57) **Abstract**

The present invention provides a method and system for diagnosing oral diseases of a companion animal. The method is a method of diagnosing oral diseases of a companion animal, which is performed by a health management server, and may comprise the steps of: generating health standard data by matching basic condition information and basic result data; receiving, from a user terminal, condition measurement information of an object to be inspected; and comparing and analyzing the condition measurement information on the basis of the health standard data to generate health result data, wherein the step of generating the health standard data comprises the steps of: preprocessing an image included in basic photographing information included in the basic condition information; extracting an analyzable analysis image from the preprocessed image; extracting, from the analysis image, site-specific analysis images for analyzing a plurality of diseases to diagnose the presence or absence of oral diseases in the site-specific analysis images, thereby generating basic diagnostic data; determining a disease-specific progression stage in the site-specific analysis images on the basis of the basic diagnostic data to generate basic determination data; and generating the basic result data including the basic diagnostic data and the basic determination data corresponding to the basic diagnostic data.

## Description

### [Technical Field]

The present invention relates to a method and system for diagnosing an oral disease of a companion animal, and more particularly, to a diagnosis method and system capable of quickly determining the presence or absence of a disease in an oral part of a companion animal, thereby enabling rapid treatment and preventing misdiagnosis.

### [Background Art]

As a population structure ages and the number of single-person households increases, humans are becoming increasingly self-centered and mentally devastated. Accordingly, the number of people who recognize their pets as family members or companions is increasing, and the companion animal market is also steadily growing.

When an unusual symptom occurs in such a companion animal, the companion animal is taken to a veterinary hospital for treatment or the unusual symptom of the companion animal is resolved based on information obtained from nearby people or through the Internet or telephone.

However, since the information obtained from nearby people or through the Internet or telephone often contains incorrect information, medical treatment may be difficult, and even when customers directly visit a hospital, long waiting times and heavy workloads often prevent the hospital from providing proper services (see Korean Laid-open Patent Publication No. 10-2021-0108686 published on September 3, 2021).

The matters described as the background technology above are provided only to facilitate understanding of the background of the present invention, and are not to be construed as an acknowledgment that the matters correspond to prior art already known to those skilled in the art.

### [Disclosure]

### [Technical Problem]

An object of the present invention is to solve is to provide a method and system for diagnosing an oral disease in a companion animal.

The object of the present invention is not limited to the object described above, and other objects that are not described will be clearly understood by those skilled in the art from the description below.

### [Technical Solution]

A method of diagnosing an oral disease of a companion animal according to an aspect of the present invention for solving the above-described problem includes generating health standard data by matching basic condition information with basic result data; receiving condition measurement information of an examination subject from a user terminal; and comparing and analyzing the condition measurement information based on the health standard data to generate health result data, wherein the generating of the health standard data includes preprocessing an image included in basic photographing information included in the basic condition information; extracting an analyzable analysis image from the preprocessed image; extracting an analysis image for each part for analyzing a plurality of diseases from the analysis image and diagnosing the presence or absence of the oral disease for the analysis image for each part to generate basic diagnosis data; determining a disease-specific progression stage for the analysis image for each part based on the basic diagnosis data to generate basic determination data; and generating the basic result data including the basic diagnosis data and the basic determination data corresponding to the basic diagnosis data.

According to another aspect of the present invention, the generating of the health result data may include extracting an actual analysis image from actual photographing information included in the condition measurement information of the examination subject; extracting an actual analysis image for each part from the actual analysis image and diagnosing the presence or absence of the oral disease for the actual analysis image for each part to generate the actual diagnosis data; determining a disease-specific progression stage for the actual analysis image for each part based on the actual diagnosis data to generate the actual determination data; and generating the health result data including the actual diagnosis data and the actual determination data corresponding to the actual diagnosis data.

According to still another aspect of the present invention, the extracting of the actual analysis image may include analyzing a degree of shaking of a first image when the actual photographing information includes the first image; checking whether the degree of shaking of the first image is smaller than a first reference value when the degree of shaking of the first image analyzed as a first value; analyzing the sharpness of the first image when the first value is checked to be lower than the first reference value; determining whether the second value is greater than the second reference value when the sharpness of the first image is analyzed as a second value; analyzing which part of the companion animal is captured in the first image when the second value is checked to be greater than the second reference value; and classifying the first image as an analyzable image and extracting the first image as the actual analysis image when the first image is determined to be an image of the oral part.

According to yet another aspect of the present invention, the generating of the actual diagnosis data may include analyzing the brightness of the first image when the first image is extracted as the actual analysis image; determining whether a third value is included within a reference range when the brightness of the first image is analyzed as the third value; determining that correction of the first image is not necessary when the third value is determined to be included within the reference range; setting a correction value for the first image based on the third value when the third value is determined to be outside the reference range; and performing brightness correction for the first image using the correction value.

According to yet another aspect of the present invention, the generating of the actual diagnosis data may include cropping a region occupied by teeth in the first image to extract the region as a second image; analyzing whether there is a symptom of at least one of cavities, tartar, tooth fracture, tooth discoloration, residual baby teeth, and tooth loss based on the second image and diagnosing the presence or absence of the oral disease in the tooth part to generate the actual diagnosis data; cropping a region occupied by gums in the first image to extract the area as a third image; and analyzing whether there is a symptom of at least one of gum inflammation and gum tumor based on the third image, and diagnosing the presence or absence of the oral disease in the gum part to generate the actual diagnosis data.

According to yet another aspect of the present invention, the generating of the basic result data may include analyzing the analysis image for each part with the naked eye based on basic information of the examination subject included in the basic condition information, and classifying the oral disease into presence and labeling the basic diagnosis data when there is tartar on teeth of the examination subject or inflammation in gums of the examination subject.

Further, a system for diagnosing an oral disease of a companion animal according to yet another aspect of the present invention for solving the above-described problem includes a user terminal configured to generate condition measurement information including actual photographing information acquired from an examination subject; and a health management server configured to repeatedly learn health standard data generated by matching basic condition information with basic result data and analyze the condition measurement information to generate health result data for the examination subject, wherein the health management server extracts an actual analysis image from the actual photographing information, analyzes an actual analysis image for each part extracted from the actual analysis image simultaneously to diagnose the presence or absence of the oral disease for the examination subject and generate actual diagnosis data, generates the health result data including actual determination data generated by determining a disease-specific progression stage of the oral disease based on the actual diagnosis data, preprocesses an image included in basic photographing information included in the basic condition information, extracts an analyzable analysis image from the image, extracts an analysis image for each part for analyzing a plurality of diseases from the analysis image and diagnosing the presence or absence of the oral disease for the analysis image for each part to generate basic diagnosis data, determines a disease-specific progression stage for the analysis image for each part based on the basic diagnosis data to generate basic determination data, and generates the basic result data including the basic diagnosis data and the basic determination data corresponding to the basic diagnosis data.

A program according to yet another aspect of the present invention is stored in a computer-readable recording medium coupled to a computer as hardware to perform the method of diagnosing an oral disease of a companion animal.

Other specific details of the present invention are included in the detailed description and drawings.

### [Advantageous Effects]

According to the present invention, when an abnormality occurs in an oral part of an examination subject, particularly a companion animal, it is possible to diagnose an oral condition of the companion animal using a portable terminal and quickly and accurately determine a current condition of the companion animal, thereby providing a sense of confidence to a guardian of the companion animal.

According to the present invention, it is possible to accurately diagnose a current condition of the companion animal in real time using a portable terminal, thereby improving the convenience and reliability for the user.

According to the present invention, it is possible to receive hospital information for a current condition of the companion animal, so that a disease of the companion animal can be treated early at a hospital capable of responding to the disease of the companion animal, thereby protecting health of the companion animal.

According to the present invention, it is possible to continuously provide notification information for the companion animal to a guardian, thereby inducing re-visit to a veterinary hospital and preventing disengagement.

According to the present invention, it is possible to share result data of the companion animal with linked services so that a condition of the companion animal can be more accurately ascertained and timely response to the condition of the companion animal can be performed, thereby providing confidence to a guardian of the companion animal.

The effects of the present invention are not limited to the effects mentioned above, and other effects that are not mentioned can be clearly understood by those skilled in the art from the description below.

### [Description of Drawings]

FIG. 1 is a conceptual diagram illustrating a system for diagnosing an oral disease of a companion animal according to an embodiment of the present invention.
FIG. 2 is a diagram illustrating a detailed configuration of the system for diagnosing an oral disease of a companion animal illustrated in FIG. 1.
FIG. 3 is a diagram illustrating labeled basic result data corresponding to basic condition information.
FIG. 4 is a diagram illustrating a method of verifying the basic result data.
FIG. 5 is a diagram illustrating a method of diagnosing an oral disease of a companion animal according to an embodiment of the present invention.
FIG. 6 is a detailed diagram illustrating a method of generating health standard data illustrated in FIG. 5.
FIG. 7 is a detailed diagram illustrating a method of generating basic result data illustrated in FIG. 6.
FIG. 8 is a diagram illustrating a process of extracting an actual analysis image according to an embodiment of the present invention.
FIG. 9 is a diagram illustrating a result of extracting the actual analysis image.
FIG. 10 is a diagram illustrating a process of processing brightness correction of an image according to an embodiment of the present invention.
FIG. 11 is a diagram illustrating a result of performing brightness correction on an image.
FIG. 12 is a diagram illustrating a process of diagnosing the presence or absence of an oral disease to generate actual diagnosis data according to an embodiment of the present invention.
FIG. 13 is a diagram illustrating a result of extracting an analysis image for each part.

### [Modes of the Invention]

The advantages and features of the present invention, and methods for achieving these will become apparent with reference to embodiments that will be described in detail below together with the accompanying drawings. However, the present invention is not limited to the embodiments that will be described hereinafter, but may be implemented in various different forms, the present embodiments are provided only to make the disclosure of the present invention complete and to fully inform those skilled in the art of the scope of the present invention, and the present invention is defined only by the claims.

The terms used herein are intended to describe the embodiments and are not intended to limit the present invention. In the present specification, singular forms include plural forms unless specifically stated otherwise. The terms "comprise" and/or "comprising" as used herein do not exclude the presence or addition of one or more other components in addition to mentioned components. The same signs refer to the same components throughout the specification, and "and/or" includes each of mentioned components and one or more combinations thereof. Although "first," "second," and the like are used to describe various components, it is obvious that these components are not limited by such terms. These terms are only used to distinguish one component from another. Therefore, it is obvious that a first component that will be mentioned hereinafter may also be a second component within the technical spirit of the present invention.

Unless otherwise defined, all terms (including technical and scientific terms) used herein may be used with meanings that can be commonly understood by those skilled in the art to which the present invention belongs. Further, terms defined in a commonly used dictionary shall not be construed ideally or excessively unless explicitly specifically defined.

Hereinafter, embodiments of the present invention will be described in detail with reference to the accompanying drawings.

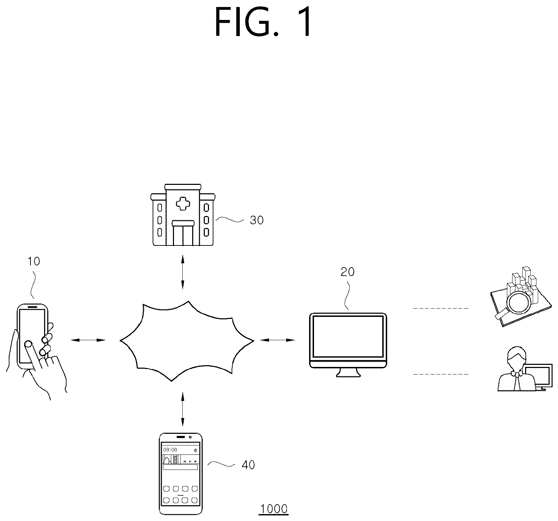

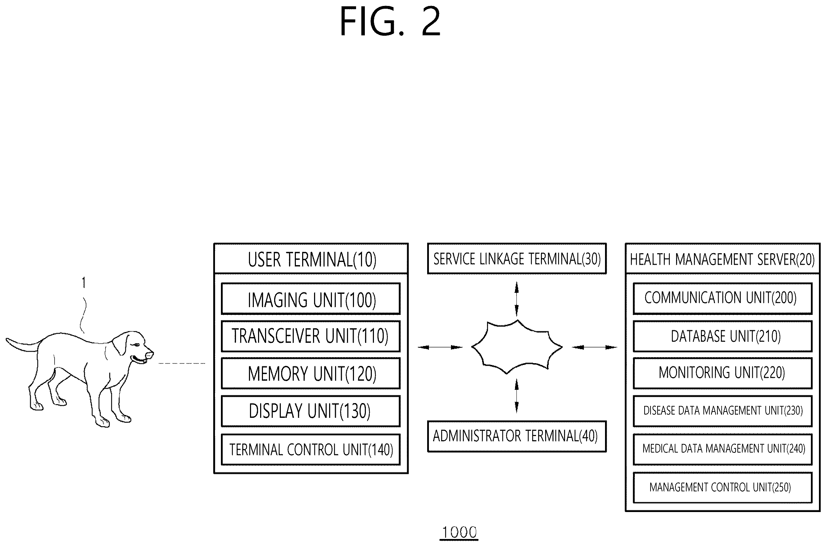

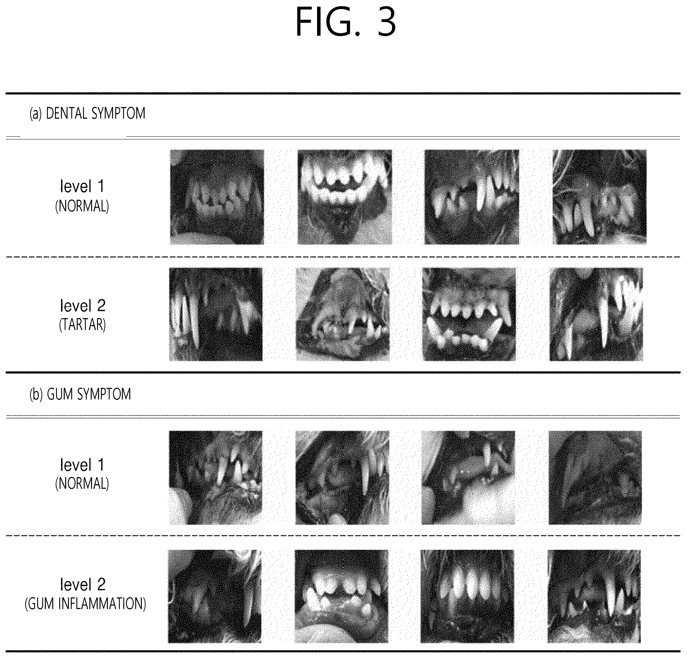

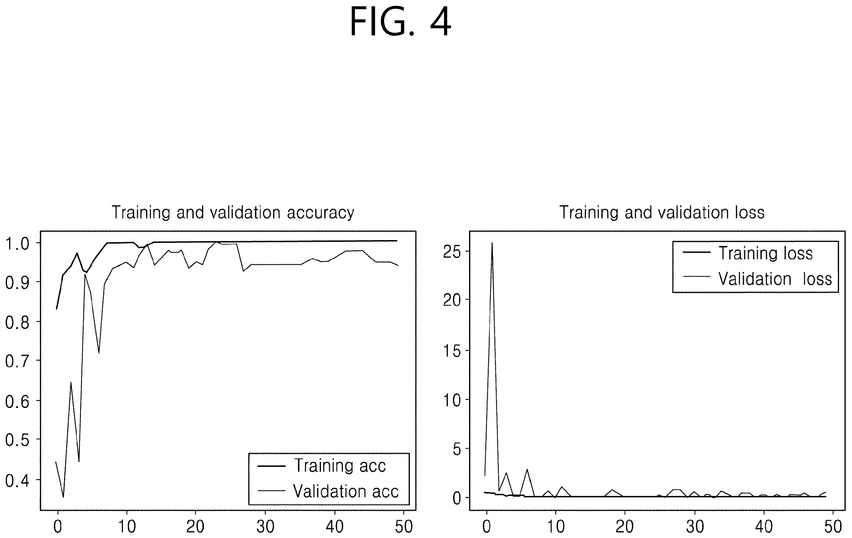

FIG. 1 is a conceptual diagram illustrating a system for diagnosing an oral disease of a companion animal according to an embodiment of the present invention, FIG. 2 is a diagram illustrating a detailed configuration of the system for diagnosing an oral disease of a companion animal illustrated in FIG. 1, FIG. 3 is a diagram illustrating labeled basic result data corresponding to basic condition information, and FIG. 4 is a diagram illustrating a method of verifying the basic result data.

As illustrated in FIGS. 1 and 2, a system 1000 for diagnosing an oral disease of a companion animal according to an embodiment of the present invention may include a user terminal 10, a health management server 20, a service linkage terminal 30, and an administrator terminal 40. In this case, the administrator terminal 40 may be omitted.

Here, the user terminal 10, the health management server 20, the service linkage terminal 30, and the administrator terminal 40 may be synchronized in real time using a wireless communication network to transmit and receive data. The wireless communication network may support various long-range communication schemes, and for example, various communication schemes such as a wireless LAN (WLAN), Digital Living Network Alliance (DLNA), wireless broadband (Wibro), World Interoperability for Microwave Access (Wimax), Global System for Mobile Communication (GSM), code division multi access (CDMA), code division multi access 2000 (CDMA2000), enhanced voice-data optimized or enhanced voice-data only (EV-DO), wideband CDMA (WCDMA), high speed downlink packet access (HSDPA), high speed uplink packet access (HSUPA), IEEE 802.16, Long Term Evolution (LTE), LTE-advanced (LTEA), wireless mobile broadband service (WMBS), Bluetooth low energy (BLE), Zigbee, radio frequency (RF), and long range (LoRa) may be applied, but the present invention is not limited thereto, and various widely known wireless communication or mobile communication schemes may be applied.

First, in the present embodiment, it is disclosed that the system 1000 for diagnosing an oral disease of a companion animal is used to photograph an oral part of the companion animal, especially a dog, determine the presence or absence of an oral disease, and determine a disease-specific progression stage when there is an oral disease, but the present invention is not limited thereto. For example, it is also possible to measure oral diseases of various animals including vertebrates such as mammals, birds, reptiles, amphibians, and fish, invertebrates such as arthropods and mollusks, and the like, living together with guardians, companions, or dog owners (hereinafter referred to as guardians), as well as oral diseases of humans.

The user terminal 10 may be a portable terminal that is carried by a guardian of the companion animal 1, the user terminal 10 may operate using an application program or application in this disclosure, and this application program may be downloaded from an external server or the health management server 20 via wireless communication. Examples of the user terminal 10 may include a smart phone, a personal digital assistant (PDA), a tablet, a wearable device (for example, a smartwatch, a smart glass, or a head mounted display (HMD)), or an Internet of Things (IoT) terminal, but is not limited thereto.

Such a user terminal 10 may include an imaging unit 100, a transceiver unit 110, a memory unit 120, a display unit 130, and a terminal control unit 140, as illustrated in FIG. 2.

The imaging unit 100 may recognize the oral part of the companion animal 1 using a camera (not shown) included in the user terminal 10 and photograph the oral part to acquire actual photographing information. Here, the actual photographing information is information generated by actually photographing the oral part of the companion animal 1, and may include information on photographs and/or videos showing a condition in which management of the companion animal 1 is required. For example, the actual photographing information may include photographs or videos showing an oral condition of the companion animal 1, but the present invention is not limited thereto.

The transceiver unit 110 may transmit the condition measurement information to the health management server 20, and may receive health management data generated based on health standard data from the health management server 20. In this case, the health standard data may be data representing a standard for the oral health of the companion animal 1 and may be updated in real time in response to the health result data.

Here, the condition measurement information may be information that is used to measure the oral condition of the companion animal 1, and may include photographs and videos whose brightness, resolution, and the like are automatically adjusted in consideration of a surrounding environment, shaking, clarity, whether or not an oral cavity is photographed, and the like, based on the actual photographing information.

Further, the health standard data may be data generated by matching the basic condition information of the companion animal 1 with the basic result data.

The basic condition information may be information indicating a basic condition of the companion animal 1, and the basic condition information may include basic information and basic photographing information. The basic information may include various types of information related to the companion animal 1, such as guardian information, abandonment information, hospital record information, a unique identification number, a dog breed, a sex, an age, a weight, a neutering status, birth history, and the like, the guardian information may include contact information and the like, and the hospital record information may include vaccination information, treatment information, presence or absence of allergies, and the like. In this case, according to the embodiment, the hospital record information may include grooming information. The basic photographing information may include information generated by photographing the companion animal 1, and may include general photographing information obtained by photographing in a general photographing mode.

The basic result data may include the basic diagnosis data and the basic determination data. The basic diagnosis data is data obtained by diagnosing the presence or absence of the oral disease using the basic photographing information, and the basic determination data may be data obtained by determining a disease-specific progression stage for the oral disease based on the basic diagnosis data. In this case, the basic diagnosis data may be determined with the naked eye, but the present invention is not limited thereto.

The health result data may include actual diagnosis data and actual determination data. The actual diagnosis data is data obtained by diagnosing the presence or absence of the oral disease of the companion animal 1 using the condition measurement information based on the health standard data, and the actual determination data may be data obtained by determining the disease-specific progression stage corresponding to the actual diagnosis data based on the health standard data. In this case, the actual diagnosis data may be determined with the naked eye, but the present invention is not limited thereto.

Here, disease names of oral diseases may include cavities, tartar, tooth fracture, tooth discoloration, residual baby teeth, tooth loss, gingivitis, gum tumor, and the like, but are not limited thereto.

According to the embodiment, the transceiver unit 110 may receive the health standard data from the health management server 20 and transmit the health result data to the health management server 20.

According to an embodiment, when the transceiver unit 110 transmits the condition measurement information from the user terminal 10 to the health management server 20, the transceiver unit 110 may receive the health result data from the health management server 20.

The transceiver unit 110 may transmit and receive medical management data. Here, the medical management data may include recommendation information that can be recommended according to the current condition or disease condition of the companion animal 1, reservation management information, veterinary hospital linkage information, and hospital record information, but is not limited thereto.

The memory unit 120 may store data transmitted and received through the transceiver unit 110 and data for supporting various functions of the user terminal 10.

The memory unit 120 may store a number of application programs (or applications) operated on the user terminal 10, data for an operation of the user terminal 10, and instructions. At least some of the application programs may be downloaded from an external server via wireless communication.

The display unit 130 is a means for visually and audibly displaying a current operation status of the user terminal 10, and may include a display unit that can output symbols, letters, numbers, and the like on a screen according to the operation status, a color-changing or blinking lamp, or a speaker that outputs audio.

For example, when the health result data is output after measurement of the companion animal 1 is completed, the display unit 130 may display the actual diagnosis data as O/X, blink the screen in red or green, display guidance text such as "Normal" or "We recommend visiting a hospital," or display the actual determination data together with predefined information. In this case, the display unit 130 may provide guidance text audibly so that the guardian can accurately check results of the companion animal 1.

Further, when the medical management data is output, hospital information such as a location, contact information, and available dates of a hospital corresponding to recommendation information generated based on the veterinary hospital linkage information may be displayed along with a road view, map, or calendar, hospital record information including treatment history information, prevention information, and the like may be displayed, or reservation management information including a reservation completion signal received in response to a reservation request signal from the user terminal 10 may be displayed on the screen.

The terminal control unit 140 may generate the condition measurement information of the companion animal 1 by operating the imaging unit 100 through a manual operation performed by the guardian, and may receive and output health result data with respect to the condition measurement information.

Specifically, the terminal control unit 140 may recognize the oral part of the companion animal 1 and automatically correct actual photographing information acquired centered on the oral cavity to generate the condition measurement information. In this case, the condition measurement information may include actual basic information and the actual photographing information. Here, the actual basic information may include guardian information, hospital record information, a unique identification number, a dog breed, a sex, an age, a weight, a neutering status, birth history, and the like, the guardian information may include contact information and the like, and the hospital record information may include vaccination information, treatment information, presence or absence of allergies, and the like. In this case, according to the embodiment, the hospital record information may include grooming information.

The condition measurement information may include photographs and videos generated by automatically adjusting brightness and clarity in consideration of a surrounding environment, shaking, clarity, whether an oral cavity is photographed, and the like from the actual photographing information. Here, the number of photographs may be at least 1, and the video may be at least 10 seconds in length, but the present invention is not limited thereto.

In other words, the terminal control unit 140 may receive the health result data for the condition measurement information regardless of time and place using a portable user terminal 10 to accurately check the presence or absence of the oral disease of the companion animal 1 and the disease-specific progression stage of the oral disease. This makes it possible to protect the health of the companion animal 1 by treating the oral disease of the companion animal 1 at an early stage, thereby improving convenience and reliability while respecting the diversity of guardians.

According to an embodiment, when the terminal control unit 140 receives the health standard data from the health management server 20, the terminal control unit 140 may compare and analyze the condition measurement information based on the health standard data to generate the health result data.

Further, the terminal control unit 140 may transmit and receive medical management data corresponding to the current condition or disease condition of the companion animal 1 based on the health result data.

Specifically, the terminal control unit 140 may generate a reservation request signal including hospital selection, a hospital reservation request, and the like according to recommendation information recommended according to health result data, based on the veterinary hospital linkage information, transmit the reservation request signal to the health management server 20 or the service linkage terminal 30, and receive a reservation completion signal corresponding to the reservation request signal from the health management server 20 or the service linkage terminal 30. Further, the terminal control unit 140 may receive hospital record information of the companion animal 1 from the health management server 20 or the service linkage terminal 30.

The health management server 20 may include a communication unit 200, a database unit 210, a monitoring unit 220, a disease data management unit 230, a medical data management unit 240, and a management control unit 250.

When the communication unit 200 receives the condition measurement information from the user terminal 10, the communication unit 200 may transmit health result data to the user terminal 10.

According to an embodiment, when the communication unit 200 transmits the health standard data to the user terminal 10, the communication unit 200 may receive the health result data from the user terminal 10.

Further, the communication unit 200 may transmit and receive the medical management data between the user terminal 10 and the health management server 20.

According to an embodiment, the communication unit 200 may transmit and receive the medical management data between the user terminal 10 and the service linkage terminal 30.

The database unit 210 may store data transmitted and received between the user terminal 10 and the service linkage terminal 30 via the wireless communication network. In this case, the health standard data may be updated and stored in real time in response to the health result data.

The database unit 210 may store data for supporting various functions of the health management server 20. The database unit 210 may store a number of application programs (or applications) operated on the health management server 20, data for an operation of the health management server 20, and instructions. At least some of the application programs may be downloaded from an external server via wireless communication.

Meanwhile, the basic condition information, the condition measurement information, the health result data, and the health standard data used in the present embodiment, which are stored in the database unit 210, may be implemented in the form of a mapping table in which data correspond to each other, but the present invention is not limited thereto.

The monitoring unit 220 may monitor an operation status of the user terminal 10, an operation status of the health management server 20, data transmitted and received between the user terminal 10 and the health management server 20, and the like through the screen. That is, a usage status of the user terminal 10 may be checked in real time to enhance the convenience for the guardian and provide more confidence to the guardian.

The disease data management unit 230 may acquire the basic condition information for a plurality of companion animals 1 and analyze the acquired basic condition information to generate the basic result data. In this case, the basic condition information may be information acquired from dogs at a facility such as an abandoned dog center or shelter, but is not limited thereto.

The disease data management unit 230 may input basic information on the plurality of companion animals 1 using a mobile terminal, set a photographing part of the companion animal 1, and acquire basic photographing information including the general photographing information captured in the general photographing mode.

In the present embodiment, a case in which the basic condition information includes basic photographing information for the oral cavity of the companion animal 1 based on the basic information for the plurality of companion animals 1 has been disclosed, but the present invention is not limited thereto and basic photographing information for various parts such as a face, eyes, a head, a stomach, feet, and a chest may be included. In this case, the basic photographing information may include a photo or a video.

For example, in order to collect basic photographing information for the oral part, the disease data management unit 230 may allow a tongue of the companion animal 1 to be accurately photographed in the general photographing mode.

Further, the disease data management unit 230 may determine whether or not there is an oral disease using the basic photographing information included in the basic condition information, and generate basic result data including the disease-specific progression stage for an oral disease when it is determined that there is an oral disease. For example, referring to FIG. 3, the disease data management unit 230 may analyze presence or absence of the oral disease using the basic photographing information, classify and label the oral disease as level 1 (normal), level 2 (tartar), and the like when the basic photographing information is information captured to diagnose a dental symptom, and classify and label the oral disease as level 1 (normal), Level 2 (gum inflammation), and the like when the basic photographing information is information captured to diagnose a gum symptom, for digitization.

Specifically, the disease data management unit 230 preprocesses the basic photographing information based on the basic information of the companion animal 1 to extract and correct an analyzable image, extracts an analysis image for each part from the extracted analysis images to diagnose the presence or absence of the oral disease diagnosable with the naked eye and generate basic diagnosis data, and simultaneously analyzes the analysis image for each part based on the basic diagnosis data to determine the disease-specific progression stage, thereby generating basic determination data obtained by analyzing a progression stage of a plurality of diseases. Here, the basic diagnosis data is data allowing abnormal signs inside the oral cavity to be diagnosed with the naked eye, and the basic determination data may be data generated by extracting the analysis image for each part for analyzing a plurality of diseases from the analysis images extracted based on the basic diagnosis data. For example, the disease data management unit 230 may determine whether there is dental calculus or gum inflammation using the analysis images for each part extracted from the teeth and gums, diagnose that there is an oral disease based on a result of the determination to generate the basic diagnosis data for the presence or absence of the oral disease, and simultaneously determine the analysis images for each part using a densely connected inception network (DCIN) algorithm based on the basic diagnosis data to more clearly generate the basic determination data including the disease-specific progression stage.

According to an embodiment, the DCIN algorithm has the advantage of reducing an amount of calculation using a 1X1 convolutional layer, extracting a feature value through parallel operation of convolutional layers having different sizes, strengthening feature propagation through reuse of feature values, and reducing overfitting with a regularizing effect of dense connection in a situation in which a data set for each case in an early stage of development is less due to the characteristics of standard shooting images.

As described above, the health management server 20 may analyze the analysis image for each part with the naked eye based on the basic information of the examination subject included in the basic condition information, classify the oral disease into presence when there is tartar on the teeth of the examination subject or inflammation on the gums of the examination subject, and label the basic diagnosis data.

The health management server 20 may perform learning and verification through an oral symptom diagnosis model based on a DCIN, which is a deep learning algorithm.

Specifically, the health management server 20 may generate a learning data set and a verification data set from the labeled data collected using a research system, perform data learning based on DCIN for optimal image characteristic extraction related to the oral disease, and then check whether overfitting occurs in learning data for each part in which oral disease characteristics appear, to verify the model suitability. In this case, the health management server 20 may perform verification by comparing extraction success rates of a training data set and a test data set, and may perform repeated learning for normalizing an image extraction part (teeth and gums) and additionally collecting the learning data set when overfitting occurs.

The disease data management unit 230 may extract an analyzable analysis image in consideration of a surrounding environment, shaking, clarity, whether or not an oral cavity is photographed, and the like from the photo or video included in the basic photographing information when the basic photographing information is information obtained by filming the oral part, automatically correct the brightness, clarity, and the like of the extracted analysis image, extract the analysis image for each part for analyzing a plurality of diseases such as teeth and gums from the corrected analysis image, analyze the analysis images simultaneously to determine a disease-specific progression stage, and label this to generate the basic result data.

Meanwhile, when the basic photographing information is a photo, the disease data management unit 230 may generate the basic diagnosis data and the basic determination data for one photo. On the other hand, when the basic photographing information is a video, the disease data management unit 230 may generate the basic diagnosis data and the basic determination data by determining normal images from the video through a filtering step and extracting at least 10 images. In this case, the normal image may be acquired by filtering a degree of shaking of the image included in the video using a Laplace filter when an area occupied by the oral part in a total area is equal to or larger than a certain area, but the present invention is not limited thereto.

The medical data management unit 240 may manage the medical management data transmitted and received between the user terminal 10 and the service linkage terminal 30 based on the health result data. Here, the medical management data may include the recommendation information that can be recommended according to the current condition or disease condition of the companion animal 1, the reservation management information, the veterinary hospital linkage information, and the hospital record information.

For example, the medical data management unit 240 may provide the user terminal 10 with recommendation information generated based on the hospital information when medical treatment is required according to the actual diagnosis data. In this case, the recommendation information may be information for recommending the hospital information corresponding to the health result data using the veterinary hospital linkage information generated based on the hospital information provided from the service linkage terminal 30.

Further, the medical data management unit 240 may transmit and receive the reservation management information between the user terminal 10 and the service linkage terminal 30.

For example, the medical data management unit 240 may transmit the reservation request signal received from the user terminal 10 to the service linkage terminal 30, and transmit a reservation completion signal generated in response to the reservation request signal from the service linkage terminal 30 to the user terminal 10.

Further, the medical data management unit 240 may transmit the hospital record information to the user terminal 10. In this case, the medical data management unit 240 may receive the hospital record information from the service linkage terminal 30.

For example, after the medical treatment of the companion animal 1 is completed, the medical data management unit 240 may transmit the hospital record information including treatment history information to the user terminal 10, and in general, may transmit the hospital record information including preventive information or grooming information to the user terminal 10. Further, the medical data management unit 240 may transmit notification information for the companion animal 1 to the user terminal 10. In this case, the notification information may be information generated by the service linkage terminal 30, which is notification information on medical treatment or grooming of the companion animal 1, but the present invention is not limited thereto.

According to the embodiment, the medical data management unit 240 may share the hospital record information with another server.

The management control unit 250 may generate the health standard data by matching the basic condition information with the basic result data using deep learning. A case in which deep learning is used has been described in the present embodiment, but the present invention is not limited thereto and a machine learning scheme such as random forest and support vector machine may be used. In this case, the management control unit 250 may update the health standard data in real time in response to the health result data.

Specifically, the management control unit 250 may repeatedly learn the basic condition information and the basic result data based on a convolutional neural network (CNN) algorithm to verify the suitability, and generate the health standard data. In this case, in a process of verifying the health standard data, the suitability may be cross-verified by at least three specialists, such as veterinarians and researchers of a commissioned research institute, as illustrated in FIG. 4, but the present invention is not limited thereto.

Further, the management control unit 250 may generate the health result data based on the health standard data when the management control unit 250 receives the condition measurement information from the user terminal 10.

Specifically, the management control unit 250 may preprocess the photographs and/or videos included in the condition measurement information and extract the actual analysis image for each image from the extracted actual analysis image to generate health result data including the actual diagnosis data allowing the presence or absence of the oral disease to be diagnosed with the naked eye and the actual determination data generated by simultaneously analyzing a plurality of diseases from the actual analysis image for each part based on the actual diagnosis data and determining the disease-specific progression stage corresponding to the actual diagnosis data.

For example, when the actual photographing information is information obtained by photographing the oral part, the management control unit 250 may extract an analyzable actual analysis image in consideration of a surrounding environment, shaking, clarity, whether or not an oral cavity is photographed, and the like from the photo or video included in the actual photographing information, automatically correct the brightness, clarity, and the like of the extracted actual analysis image, extract actual analysis image for each part for analyzing a plurality of diseases such as teeth, gums, and the like from the corrected actual analysis image, diagnose the presence or absence of the oral disease that may be diagnosed with the naked eye, analyze the actual diagnosis data, and determine the disease-specific progression stage based on the actual diagnosis data to generate actual determination information. That is, the management control unit 250 may analyze a region occupied by the tooth part in the actual analysis image to check color change, the presence or absence of wounds, and the like, and analyze a region occupied by the gum part in the actual analysis image to check color change, the presence or absence of inflammation, and the like, thereby simultaneously analyzing diseases for a plurality of parts to analyze the disease-specific progression stages of the plurality of diseases through the analysis image for each part.

Meanwhile, when the actual photographing information is a photo, the management control unit 250 may generate the actual diagnosis data and the actual determination data for one photo. On the other hand, when the actual photographing information is a video, the management control unit 250 may generate the actual diagnosis data and the actual determination data by determining normal images from the video through a filtering step and extracting at least 10 images. In this case, the normal image may be acquired by filtering a degree of shaking of the image included in the video using a Laplace filter when an area occupied by the oral part in a total area is equal to or larger than a certain area, but the present invention is not limited thereto.

According to an embodiment, when the management control unit 250 transmits the health standard data to the user terminal 10, the management control unit 250 may receive the health result data corresponding to the condition measurement information of the companion animal 1.

According to an embodiment, the management control unit 250 may insert advertising information into data transmitted to and received from the user terminal 10, the service linkage terminal 30, and/or the administrator terminal 40. Accordingly, separate advertising revenue may be generated and may be donated to facilities such as abandoned dog centers and shelters.

The health management server 20 having such a structure may repeatedly learn labeled basic result data corresponding to the basic condition information acquired from the plurality of companion animals 1, automatically extract the diagnosis part from the condition measurement information acquired through the user terminal 10 based on the verified health standard data, compare and analyze the extracted images to analyze a plurality of diseases, and generate health result data including the presence or absence of the oral disease and the disease-specific progression stage. This can resolve problems such as unnecessary hospital visits and lack of appropriate care that may occur when the condition of the companion animal 1 is determined only with the naked eye.

Further, the health management server 20 may provide the recommendation information to the user terminal 10 depending on the current condition or disease condition of the companion animal 1 to quickly and accurately manage the companion animal 1.

This health management server 20 may be implemented by a hardware circuit (for example, a CMOS-based logic circuit), firmware, software, or a combination thereof. For example, the health management server 20 may be implemented by utilizing transistors, logic gates, and electronic circuits in the form of various electrical structures.

The service linkage terminal 30 may allow the companion animals 1 to be treated more quickly by using health result data received from a plurality of veterinary hospitals that manage the health of the companion animals 1 and medically treat the companion animal 1.

The service linkage terminal 30 may share the hospital record information with the user terminal 10, the health management server 20, and a separate server.

The service linkage terminal 30 may provide the hospital information and the notification information to the user terminal 10 and/or health management server 20.

According to the embodiment, the service linkage terminal 30 may include a separate facility such as an abandoned dog center or shelter.

The administrator terminal 40 may be a terminal carried by a separate administrator, and may be synchronized with the user terminal 10, the health management server 20, and the service linkage terminal 30 in real time using the wireless communication network to transmit and receive data. In this case, the administrator terminal 40 may transmit and receive data using an application program (or application).

The administrator terminal 40 learns the health standard data received from the health management server 20, analyzes the condition measurement information received from the user terminal 10, and generates the health result data including the actual diagnosis data and the actual determination data.

According to an embodiment, when the administrator terminal 40 receives the condition measurement information from the user terminal 10, the administrator terminal 40 may compare and analyze the condition measurement information based on the health standard data to generate the health result data.

According to an embodiment, when health result data is generated in the user terminal 10, the administrator terminal 40 may receive the health result data from the user terminal 10 and transmit the health result data to the health management server 20. Further, when health result data is generated in the health management server 20, the administrator terminal 40 may receive the health result data from the health management server 20 and transmit the health result data to the user terminal 10.

According to an embodiment, the administrator terminal 40 may transmit and receive medical management data corresponding to a current condition or disease condition of the companion animal 1 based on the health result data to at least one of the user terminal 10, the health management server 20, and the service linkage terminal 30.

Such an administrator terminal 40 may include various portable electronic communication devices that support communication with the user terminal 10, the health management server 20, and the service linkage terminal 30. Examples of a separate smart device may include a smart phone, a personal digital assistant (PDA), a tablet, a wearable device (for example, a smartwatch, a smart glass, or a head mounted display (HMD)), and any Internet of Things (IoT) terminal, but the present invention is not limited thereto.

An operation of the system for diagnosing an oral disease of a companion animal according to an embodiment of the present invention having such a structure is as follows.

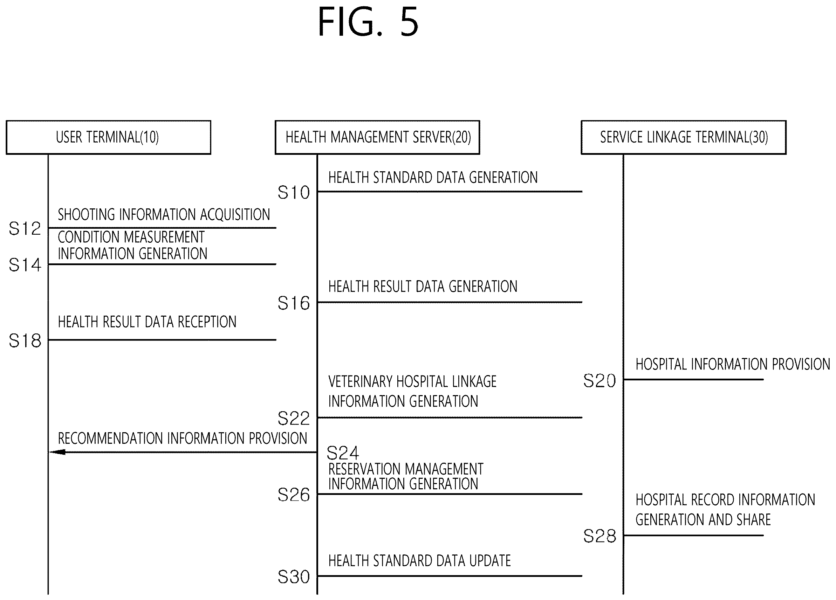

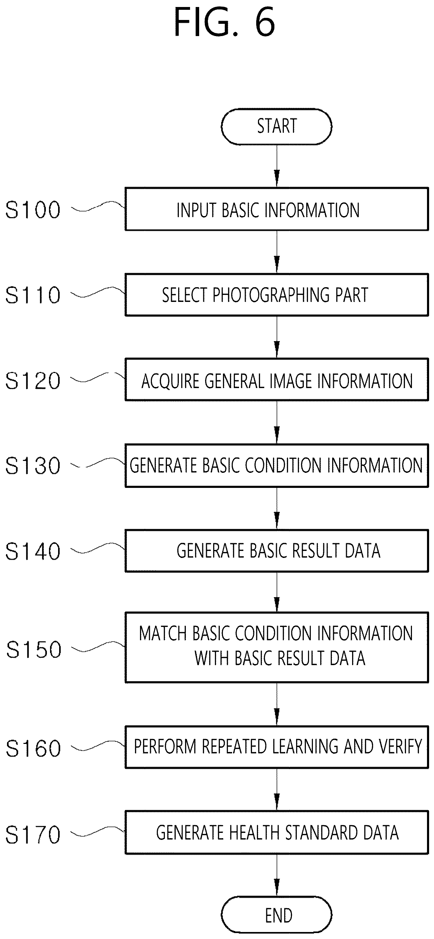

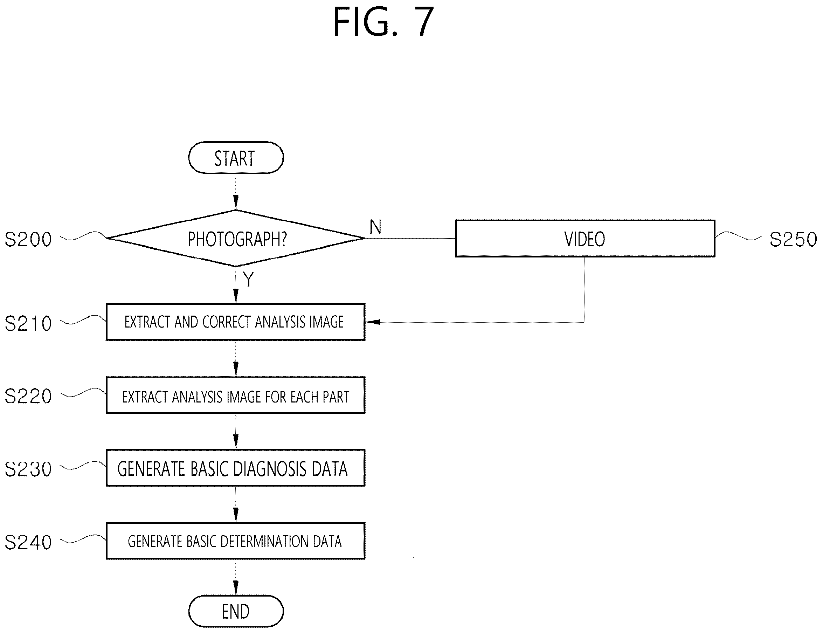

FIG. 5 is a diagram illustrating a method of diagnosing an oral disease of a companion animal according to an embodiment of the present invention, FIG. 6 is a detailed diagram illustrating a method of generating the health standard data illustrated in FIG. 5, and FIG. 7 is a detailed diagram illustrating a method of generating basic result data illustrated in FIG. 6.

First, the embodiment of the present invention discloses that the companion animal 1 is a dog, but the present invention is not limited thereto.

As illustrated in FIG. 5, the health management server 20 may generate the health standard data (S10).

Specifically, referring to FIG. 6, the health management server 20 may acquire the basic information on the plurality of companion animals 1 (S100). Here, the basic information may include guardian information, abandonment information, hospital record information, a unique identification number, a dog breed, a sex, an age, a weight, a neutering status, and birth history, but the present invention is not limited thereto.

For example, the disease data management unit 230 may acquire the basic information on the companion animal 1 input using a separate mobile terminal.

Next, the health management server 20 may select a photographing part of the companion animal 1 based on the basic information (S110).

For example, the disease data management unit 230 may check the photographing part of the companion animal 1 set through a separate mobile terminal. That is, the disease data management unit 230 may select various parts, such as an oral cavity, a face, ears, a stomach, feet, a chest, and a back, from body parts of the companion animal 1.

Next, when the health management server 20 photographs the oral part, the health management server 20 may first photograph the oral part of the companion animal 1 in the general photographing mode to acquire the general photographing information (S120).

For example, when the oral part of the companion animal 1 is photographed in the general photographing mode using a separate mobile terminal, the disease data management unit 230 may acquire the general photographing information from the mobile terminal.

Next, the health management server 20 may generate basic condition information using the basic photographing information including the basic information and general photographing information corresponding thereto (S130). In this case, the general photographing information may include at least one photo and a video may be at least 10 seconds in length.

Next, the health management server 20 may extract an analyzable analysis image from the basic photographing information to generate the basic result data (S140).

Specifically, as illustrated in FIG. 7, when the information included in the basic photographing information is a photograph (S200), the health management server 20 may verify whether the photograph is an analyzable image and extract an analysis image (S210).

For example, the disease data management unit 230 may extract an analyzable analysis image in consideration of a surrounding environment, shaking, clarity, whether or not an oral cavity is photographed, and the like. In this case, the extracted analysis image may be corrected. That is, the disease data management unit 230 may extract an oral part from the analysis image and correct white balance and brightness.

Next, the health management server 20 may extract the analysis image for each part for analyzing a plurality of diseases from the extracted analysis image (S220).

For example, the disease data management unit 230 may extract the analysis image for each part with respect to the tooth part from the analysis image and extract the analysis image for each part with respect to the gum part, to analyze the plurality of diseases for each part at the same time.

Next, the basic diagnosis data may be generated by diagnosing the presence or absence of the oral disease using the analysis image for each part (S230).

For example, the disease data management unit 230 may use the analysis image for each part extracted from the teeth and the gums to determine whether there is tartar or gum inflammation, and diagnose the presence of the oral disease based on results of the determination to generate the basic diagnosis data regarding the presence or absence of the oral disease.

Next, the health management server 20 may determine the analysis image for each part for analyzing the plurality of diseases based on the basic diagnosis data to generate the basic determination data (S240).

Specifically, the disease data management unit 230 may analyze the plurality of diseases through the analysis image for each part by determining color change, presence or absence of wounds, and the like in images of the tooth part, determining color change, presence or absence of inflammation, and the like in images of the gum part, and simultaneously analyzing a plurality of diseases for each part.

In this case, the disease data management unit 230 may more clearly generate the basic determination data obtained by analyzing the plurality of diseases by simultaneously determining the analysis image for each part using the DCIN algorithm based on the basic diagnosis data, but the present invention is not limited thereto.

Meanwhile, when the information included in the basic photographing information is a video (S250), opening and closing of a mouth in the video may be filtered to extract a normal image.

For example, the disease data management unit 230 may determine normal images from the video through a filtering step and extracting at least 10 images. In this case, the normal image may be acquired by filtering a degree of shaking of the image included in the video using a Laplace filter when an area occupied by the oral part in a total area is equal to or larger than a certain area.

Thus, the health management server 20 may generate the basic result data corresponding to the basic condition information.

Next, the health management server 20 may match the basic condition information with the basic result data (S150), and perform repeated learning based on a CNN algorithm to verify the suitability and generate the health standard data (S160 and S170).

Next, when the guardian requests a diagnosis of the current condition or disease condition of the companion animal 1, the user terminal 10 may film the oral part of the companion animal 1 to generate actual photographing information (S12).

Next, when actual basic information is input, the user terminal 10 may generate the condition measurement information using the actual basic information and the actual photographing information (S14).

Next, the health management server 20 may generate the health result data corresponding to the condition measurement information based on the health standard data (S16).

Specifically, the management control unit 250 may preprocess the photographs and/or videos included in the condition measurement information to extract and correct the actual analysis image, and extract the actual analysis image for each part from the corrected actual analysis image to generate health result data including the actual diagnosis data allowing the presence or absence of the oral disease for the actual analysis image for each part to be diagnosed with the naked eye, and the actual determination data generated by determining the disease-specific progression stage corresponding to the actual diagnosis data.

Next, the user terminal 10 may receive the health result data corresponding to the condition measurement information from the health management server 20 (S18).

For example, when the health result data is output after the measurement of the oral disease for the companion animal 1 is completed, the display unit 130 may output a measurement result for the oral disease on the screen.

Next, the service linkage terminal 30 may provide the hospital information based on the health standard data (S20).

Here, the step of providing the hospital information may be performed in advance, but is not limited thereto.

Next, the health management server 20 may generate the veterinary hospital linkage information based on the hospital information (S22).

Here, the step of generating the veterinary hospital linkage information may be performed in advance, but is not limited thereto.

Next, the health management server 20 may generate the recommendation information corresponding to the health result data based on the veterinary hospital linkage information and transmit the recommendation information to the user terminal 10 (S24).

Next, the health management server 20 may generate reservation management information (S26).

For example, the health management server 20 may receive a reservation request signal generated according to customized information from the user terminal 10, receive reservation management information corresponding to the reservation request signal from the service linkage terminal 30, and transmit the reservation management information to the user terminal 10.

Next, the service linkage terminal 30 may generate and share the hospital record information including the treatment history information for the companion animal 1 (S28).

In this case, the hospital record information may be transmitted to the user terminal 10 and the health management server 20.

Finally, the health management server 20 may update the health standard data in real time in response to the health result data (S30).

As described above, the system 1000 for diagnosing an oral disease of a companion animal may include the user terminal 10 and the health management server 20, the user terminal 10 may generate the condition measurement information including actual photographing information acquired from the examination subject, and the health management server 20 may repeatedly learn the health standard data generated by matching the basic condition information with the basic result data to analyze the condition measurement information and generate health result data for the examination subject.

The health management server 20 may extract the actual analysis image from the actual photographing information, simultaneously analyze the actual analysis image for each part extracted from the actual analysis image to diagnose the presence or absence of the oral disease in the examination subject and generate the actual diagnosis data, and generate health result data including the actual determination data generated by determining the disease-specific progression stage of the oral disease based on the actual diagnosis data.

The health management server 20 may preprocess an image included in the basic photographing information included in the basic condition information, extract an analyzable analysis image from the image, extract an analysis image for each part for analyzing a plurality of diseases from the analysis image to diagnose the presence or absence of the oral disease in the analysis image for each part and generate basic diagnosis data, determine a disease-specific progression stage for the analysis image for each part based on the basic diagnosis data to generate basic determination data, and generate the basic result data including the basic diagnosis data and the basic determination data corresponding to the basic diagnosis data.

That is, the health management server 20 may match the basic condition information with the basic result data to generate the health standard data, receive the condition measurement information of the examination subject from the user terminal 10, and compare and analyze the condition measurement information based on the health standard data to generate the health result data.

When the health management server 20 generates the health standard data, the health management server 20 may preprocess the image included in the basic photographing information included in the basic condition information, extract an analyzable analysis image from the preprocessed image, extract an analysis image for each part for analyzing a plurality of diseases from the analysis image to diagnose the presence or absence of the oral disease in the analysis image for each part and generate basic diagnosis data, determine a disease-specific progression stage for the analysis image for each part based on the basic diagnosis data to generate basic determination data, and generate the basic result data including the basic diagnosis data and the basic determination data corresponding to the basic diagnosis data.

When the health management server 20 generates the health result data, the health management server 20 may extract the actual analysis image from the actual photographing information included in the condition measurement information of the examination subject, extract the actual analysis image for each part from the actual analysis image, diagnose the presence or absence of the oral disease for the actual analysis image for each part to generate the actual diagnosis data, determine the disease-specific progression stage for the actual analysis image for each part based on the actual diagnosis data to generate the actual determination data, and generate the health result data including the actual diagnosis data and the actual determination data corresponding to the actual diagnosis data.

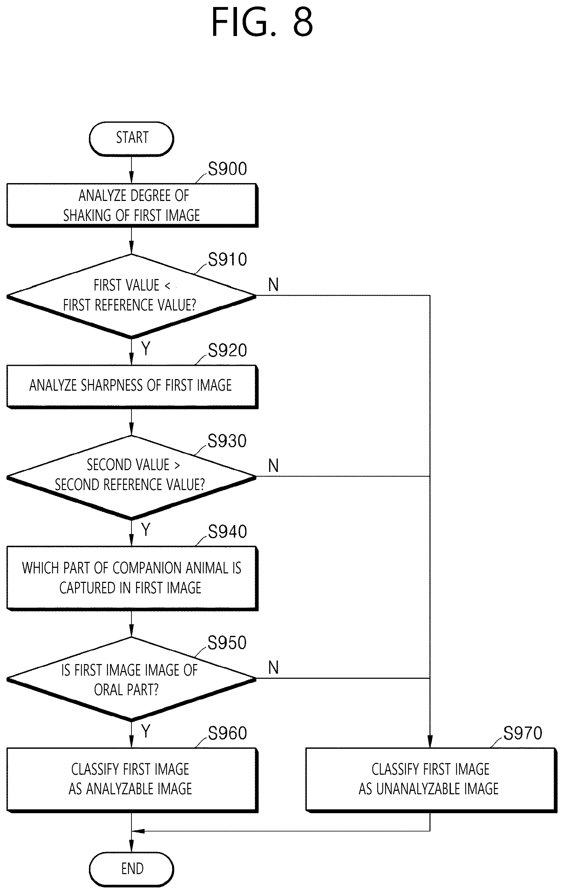

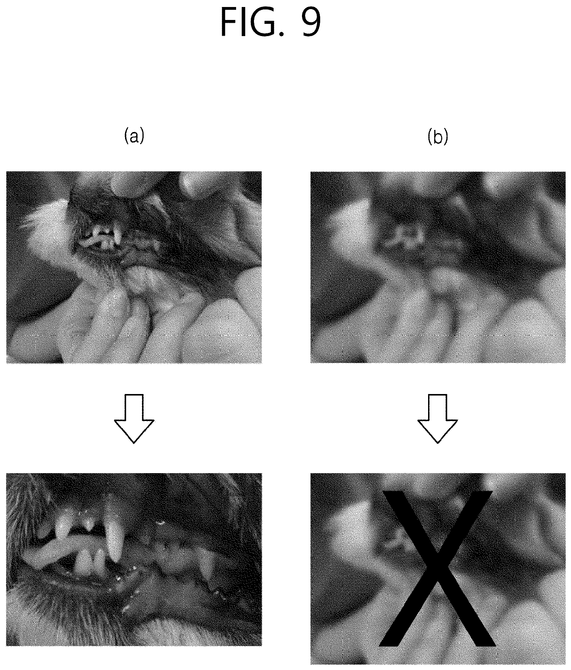

Hereinafter, a process of extracting the actual analysis image will be described in detail with reference to FIGS. 8 and 9.

FIG. 8 is a diagram illustrating a process of extracting the actual analysis image according to an embodiment of the present invention, and FIG. 9 is a diagram illustrating the result of extracting the actual analysis image.

As illustrated in FIG. 8, the health management server 20 may analyze the degree of shaking of the first image when the actual photographing information includes the first image (S900).

Specifically, the user terminal 10 may photograph the companion animal 1 to generate actual photographing information including the first image, generate the condition measurement information using the actual photographing information, and transmit the condition measurement information to the health management server 20. In this case, the health management server 20 may receive the condition measurement information from the user terminal 10, check that the actual photographing information includes the first image based on the condition measurement information, and analyze the degree of shaking of the checked first image.

When the health management server 20 analyzes the degree of shaking of the first image, the health management server 20 may analyze the degree of shaking as a shaking value indicating how much the first image shakes within a certain range, in which a greater shaking value may indicate more shaking, and a smaller value may indicate less shaking.

For example, when the degree of shaking is set within a range from 1 to 10, the health management server 20 may analyze the degree of shaking of the first image, determine that the degree of shaking of the first image is between 8 and 10 when a shaking state of the first image is a heavily shaken state, determine that the degree of shaking of the first image is between 4 and 7 when the shaking state of the first image is a moderately shaken state, and determine that the degree of shaking of the first image is between 1 and 3 when the shaking state of the first image is an almost unshaken state.

The health management server 20 may compare an image learned for each shaking value with the first image to analyze which value the degree of shaking of the first image corresponds to, and to this end, the image learned for each shaking value may be stored and managed in the database unit 210.

That is, the health management server 20 may analyze the degree of shaking of the first image and analyze the degree of shaking of the first image as the first value according to the shaking state of the first image.

When the degree of shaking of the first image is analyzed as the first value, the health management server 20 may check whether the first value is smaller than a first reference value (S910). Here, the first reference value may be set differently in some embodiments.

When the health management server 20 checks that the first value is smaller than the first reference value, the health management server 20 may analyze the sharpness of the first image (S920).

When the health management server 20 analyzes the sharpness of the first image, the health management server 20 may analyze the sharpness as a sharpness value indicating how sharp the first image is within a certain range, with sharpness having a great value indicating higher clarity and sharpness having a smaller value indicating lower clarity.

For example, when the sharpness is set within a range from 1 to 10, the health management server 20 analyzes the sharpness of the first image as one of values 8 to 10 when the first image is very sharp, analyzes the sharpness of the first image as one of values 4 to 7 when the first image is moderately sharp, and analyzes the sharpness of the first image as one of values 1 to 3 when the first image is not sharp.

The health management server 20 may compare an image learned for each sharpness value with the first image to analyze which value the sharpness of the first image corresponds to, and to this end, the image learned for each sharpness value may be stored and managed in the database unit 210.

That is, the health management server 20 may analyze the sharpness of the first image to analyze the sharpness of the first image as a second value according to the sharpness of the first image.

The health management server 20 may check whether the second value is higher than a second reference value when the sharpness of the first image is analyzed as the second value (S930). Here, the second reference value may be set differently in some embodiments.

When the health management server 20 checks that the second value is greater than the second reference value, the health management server 20 may determine which part of the companion animal is captured in the first image (S940).

Specifically, when a region occupied by a specific part in the first image is equal to or greater than a certain value, the health management server 20 may perform analysis using an image of the corresponding part. For example, when a region occupied by the oral part in the first image is found to be 70% or more as a result of analyzing which part is captured in the first image, the first image may be determined to be the image of the oral part.

The health management server 20 may check whether the first image is the image of the oral part (S950).

The health management server 20 may classify the first image as an analyzable image when the first image is determined to be the image of the oral part (S960).

The health management server 20 may classify the first image as an unanalyzable image when it is determined that the first value is not smaller than the first reference value, the second value is not greater than the second reference value, or the first image is determined to be an image of another part rather than the image of the oral part (S970).

As described above, photographs created by photographing the companion animal 1 may be classified into unanalyzable data and analyzable data based on shaking, clarity, whether teeth or gums are photographed, and the like, a process of checking whether an image is an analyzable image based on shaking, clarity, whether teeth or gums are photographed, and the like may be learned through machine learning, and the analyzable image may be cropped around the teeth and gums and then extracted as the actual analysis image.

For example, referring to FIG. 9A, the health management server 20 may classify the first image as an analyzable image based on shaking, clarity, whether or not the oral cavity is photographed, and the like for the first image illustrated in FIG. 9A, and when the first image is classified as the analyzable image, the first image may be cropped around the oral part, and then extracted as the actual analysis image.

For example, referring to FIG. 9B, the health management server 20 may classify the first image as an unanalyzable image through shaking, clarity, whether or not the oral cavity is photographed, and the like in the first image shown in FIG. 9B, and delete the first image and transmit a re-photographing request notification message to the user terminal 10 when the first image is classified as the unanalyzable image.

The health management server 20 may extract the first image as the actual analysis image and then correct the white balance and brightness of the first image.

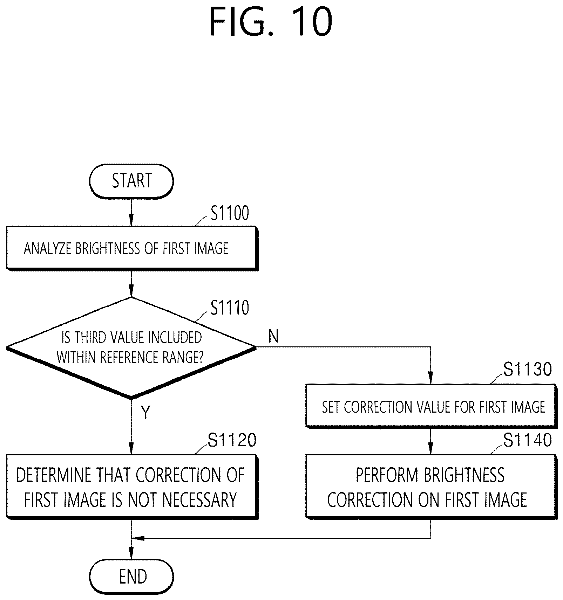

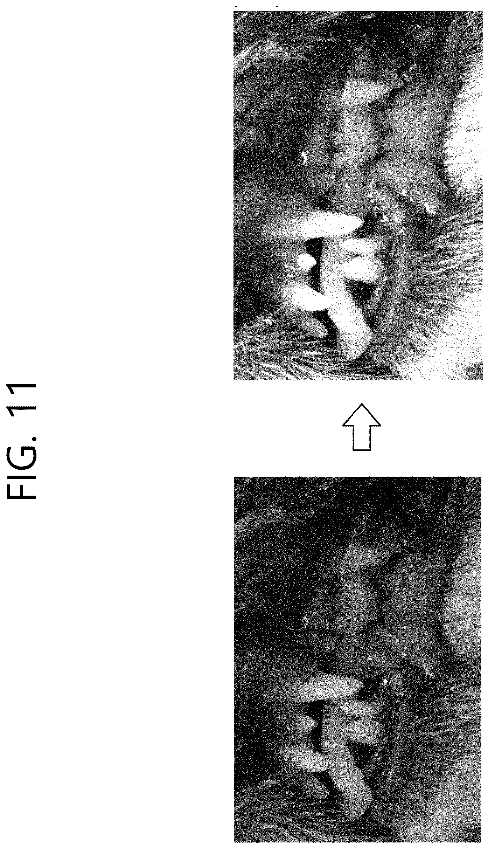

Hereinafter, a process for processing brightness correction of an image will be described in detail with reference to FIGS. 10 and 11.

FIG. 10 is a diagram illustrating a process for processing brightness correction of an image according to an embodiment of the present invention, and FIG. 11 is a diagram illustrating a result of performing the brightness correction on an image.

As illustrated in FIG. 10, the health management server 20 may analyze the brightness of the first image when the first image is extracted as the actual analysis image (S1100).

When the health management server 20 analyzes the brightness of the first image, the health management server 20 may analyze the brightness as a brightness value indicating how bright the first image is within a certain range, with brightness having a great value indicating a brighter image and brightness having a smaller value indicating a darker image.

For example, when the brightness is set within a range from 1 to 10, the health management server 20 analyzes the brightness of the first image as one of values 8 to 10 when the first image is very bright, analyzes the brightness of the first image as one of values 4 to 7 when the first image is moderately bright, and analyzes the brightness of the first image as one of values 1 to 3 when the first image is dark.

The health management server 20 may compare an image learned for each brightness value with the first image to analyze which value the brightness of the first image corresponds to, and to this end, the image learned for each brightness value may be stored and managed in the database unit 210.

That is, the health management server 20 may analyze the brightness of the first image to analyze the brightness of the first image as a third value according to the brightness of the first image.

When the brightness of the first image is analyzed as the third value, the health management server 20 may determine whether the third value is included within a reference range (S1110). Here, the reference range may be set differently in some embodiments.

When it is checked that the third value is within the reference range, the health management server 20 may determine that correction of the first image is not necessary (S1120).

For example, when the reference range is set to a range from 4 to 7 and it is determined that the third value is 5, the health management server 20 may determine that the third value is within the reference range, and thus correction of the first image is not necessary.

When it is determined that the third value is outside the reference range, the health management server 20 may set a correction value for the first image based on the third value (S1130).

When it is determined that the third value is greater than a maximum value of the reference range, the health management server 20, when setting the correction value for the first image, may set the correction value to a smaller value as the third value is greater. In this case, the correction value may be set to a value smaller than 0 to correct the image to be darker.

For example, when the reference range is set to a range from 4 to 7, the health management server 20 may set the correction value for the first image to -1 when the third value is determined to be 8, and may set the correction value for the first image to -2 when the third value is determined to be 9.

When it is determined that the third value is smaller than a minimum value of the reference range, the health management server 20, when setting the correction value for the first image, may set the correction value to a greater value as the third value is smaller. In this case, the correction value may be set to a value greater than 0 to correct the image to be brighter.

For example, when the reference range is set to a range from 4 to 7, the health management server 20 may set the correction value for the first image to 1 when the third value is determined to be 3, and may set the correction value for the first image to 2 when the third value is determined to be 2.

When the correction value for the first image is set, the health management server 20 may perform brightness correction on the first image using the correction value (S1140).

When the correction value for the first image is negative, the health management server 20 may perform brightness correction to make the first image darker using the correction value.

For example, when the correction value for the first image is determined to be -1, the health management server 20 may perform brightness correction to make the first image one level darker, and when the correction value for the first image is determined to be -2, the health management server 20 may perform brightness correction to make the first image two levels darker.

When the correction value for the first image is positive, the health management server 20 may perform brightness correction to make the first image brighter using the correction value.

For example, when the correction value for the first image is determined to be 1, the health management server 20 may perform brightness correction to make the first image one level brighter, and when the correction value for the first image is determined to be 2, the health management server 20 may perform the brightness correction to make the first image two levels brighter.

As described above, the white balance and brightness correction for the first image may be performed according to a brightness status of the first image to correct the first image to an image suitable for analysis.

For example, referring to FIG. 11, when the brightness status of the first image is dark as shown on the left, brightness correction for the first image may be performed to change the first image to an image suitable for analysis in diagnosing the oral disease as shown on the right.

After performing the brightness correction on the first image, the health management server 20 may diagnose the presence or absence of the oral disease to generate the actual diagnosis data.

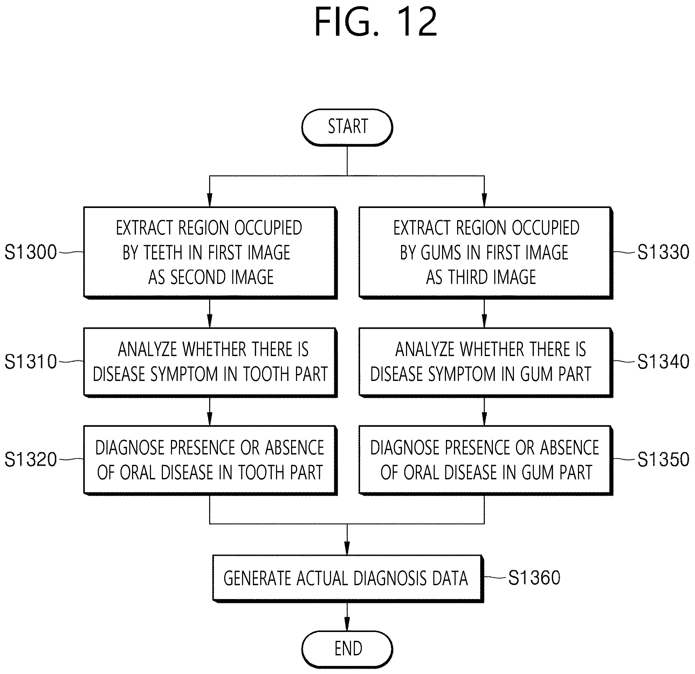

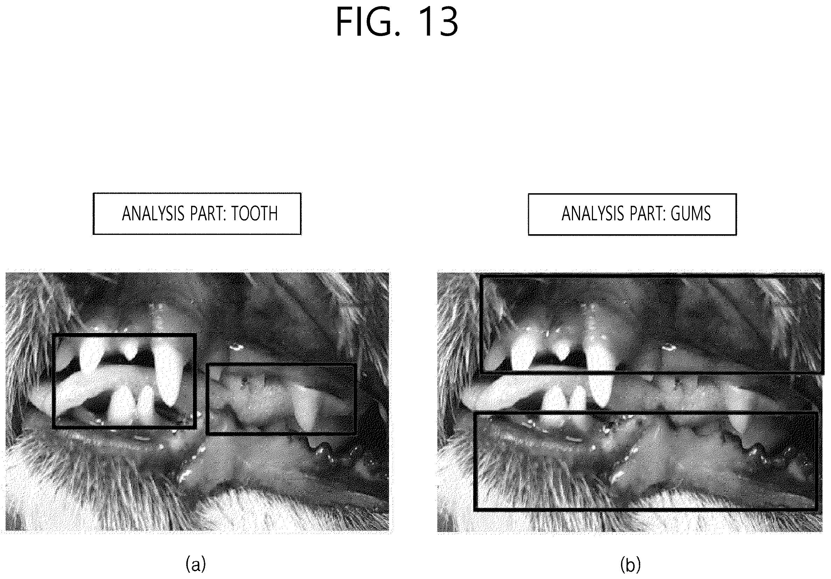

Hereinafter, a process of diagnosing the presence or absence of the oral disease to generate the actual diagnosis data will be described in detail with reference to FIGS. 12 and 13.

FIG. 12 is a diagram illustrating a process of diagnosing the presence or absence of the oral disease to generate the actual diagnosis data according to an embodiment of the present invention, and FIG. 13 is a diagram illustrating results of extracting the analysis image for each part.

As illustrated in FIG. 12, the health management server 20 may crop a region occupied by the teeth in the first image to extract the region as a second image (S1300). A CNN landmark algorithm may be applied in a process of extracting the second image.

For example, referring to FIG. 13A, when the health management server 20 analyzes the first image for each region and checks that an area occupied by the teeth in a specific region is equal to or larger than a certain area, the health management server 20 may crop the region occupied by the teeth and extract the cropped part as the second image. In this case, when there are a plurality of regions occupied by the teeth, the plurality of regions may be cropped and extracted as the second image.

The health management server 20 may analyze whether there is a disease symptom in the tooth part based on the second image (S1310). In this case, the health management server 20 may determine whether there is a symptom of at least one of cavities, tartar, tooth fracture, tooth discoloration, residual baby teeth, and tooth loss based on the second image.

The health management server 20 may analyze whether there is a disease symptom in the tooth part of the companion animal 1 by comparing the image learned for each disease symptom in the tooth part with the second image, and to this end, the image learned for each disease symptom in the tooth part may be stored and managed in the database unit 210.

The health management server 20 may diagnose the presence or absence of the oral disease in the tooth part by determining whether there is a symptom of at least one of cavities, tartar, tooth fracture, tooth discoloration, residual baby teeth, and tooth loss based on the second image (S1320).

When the health management server 20 determines that there is a symptom of at least one of cavities, tartar, tooth fracture, tooth discoloration, residual baby teeth, and tooth loss, the health management server 20 may determine the presence of the oral disease in the tooth part, and when the health management server 20 determines that there is no symptom among cavities, tartar, tooth fracture, tooth discoloration, residual baby teeth, and tooth loss, the health management server 20 may diagnose the absence of the oral disease in the tooth part.

Meanwhile, the health management server 20 may crop the region occupied by the gum part in the first image and extract the region as a third image (S1330). A CNN landmark algorithm may be applied in the process of extracting the third image.

For example, referring to FIG. 13B, when the health management server 20 analyzes the first image for each region and checks that the region occupied by the gum part in a specific region is equal to or larger than a certain area, the health management server 20 may crop the region as the region occupied by the gums and extract the cropped part as the third image. In this case, when there are a plurality of regions occupied by the gums, the plurality of regions may be cropped and extracted as the third image.

The health management server 20 may analyze whether there is a disease symptom in the gum part based on the third image (S1340). In this case, the health management server 20 may determine whether there is a symptom of at least one of gum inflammation and gum tumors based on the third image.

The health management server 20 may analyze whether there is a disease symptom in the gum part of the companion animal 1 by comparing the image learned for each disease symptom in the gum part with the third image, and to this end, the image learned for each disease symptom in the gum part may be stored and managed in the database unit 210.

The health management server 20 may diagnose the presence or absence of the oral disease in the gum part by analyzing whether there is a symptom of at least one of gum inflammation and gum tumor based on the third image (S1350).

When the health management server 20 analyzes that there is a symptom of at least one of the gum inflammation and the gum tumor, the health management server 20 may diagnose the presence of the oral disease in the gum part, and when the health management server 20 analyzes that there is no symptom of gum inflammation or gum tumor, the health management server 20 may diagnose the absence of the oral disease in the gum part.

When the presence or absence of the oral disease in each of the tooth part and the gum part is diagnosed, the health management server 20 may generate actual diagnosis data including each diagnosis result (S1360).

The operations of the method or algorithm described in relation to the embodiments of the present invention may be implemented directly by hardware, implemented by a software module executed by hardware, or implemented by a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or any other form of computer-readable recording medium well known in the art to which the present invention pertains.

Although the embodiments of the present invention have been described above with reference to the accompanying drawings, it will be understood by those skilled in the art that it is possible to implement the present invention in other specific forms without changing the technical spirit or essential features of the present invention. Therefore, the embodiments described above should be understood as illustrative in all respects and not as restrictive.

## Claims

1. A method of diagnosing an oral disease of a companion animal performed by a health management server, the method comprising:
generating health standard data by matching basic condition information with basic result data;
receiving condition measurement information of an examination subject from a user terminal; and
comparing and analyzing the condition measurement information based on the health standard data to generate health result data,
wherein the generating of the health standard data includes
preprocessing an image included in basic photographing information included in the basic condition information;
extracting an analyzable analysis image from the preprocessed image;
extracting an analysis image for each part for analyzing a plurality of diseases from the analysis image and diagnosing the presence or absence of the oral disease for the analysis image for each part to generate basic diagnosis data;
determining a disease-specific progression stage for the analysis image for each part based on the basic diagnosis data to generate basic determination data; and
generating the basic result data including the basic diagnosis data and the basic determination data corresponding to the basic diagnosis data.

2. The method of claim 1, wherein the generating of the health result data includes
extracting an actual analysis image from actual photographing information included in the condition measurement information of the examination subject;
extracting an actual analysis image for each part from the actual analysis image and diagnosing the presence or absence of the oral disease for the actual analysis image for each part to generate the actual diagnosis data;
determining a disease-specific progression stage for the actual analysis image for each part based on the actual diagnosis data to generate the actual determination data; and
generating the health result data including the actual diagnosis data and the actual determination data corresponding to the actual diagnosis data.

3. The method of claim 2, wherein the extracting of the actual analysis image includes
analyzing a degree of shaking of a first image when the actual photographing information includes the first image;
checking whether the degree of shaking of the first image is smaller than a first reference value when the degree of shaking of the first image analyzed as a first value;
analyzing a sharpness of the first image when the first value is checked to be lower than the first reference value;
determining whether the second value is greater than the second reference value when the sharpness of the first image is analyzed as a second value;
analyzing which part of the companion animal is captured in the first image when the second value is checked to be greater than the second reference value; and
classifying the first image as an analyzable image and extracting the first image as the actual analysis image when the first image is determined to be an image of the oral part.

4. The method of claim 3, wherein the generating of the actual diagnosis data includes
analyzing a brightness of the first image when the first image is extracted as the actual analysis image;
determining whether a third value is included within a reference range when the brightness of the first image is analyzed as the third value; determining that correction of the first image is not necessary when the third value is determined to be included within the reference range;
setting a correction value for the first image based on the third value when the third value is determined to be outside the reference range; and
performing brightness correction for the first image using the correction value.

5. The method of claim 4, wherein the generating of the actual diagnosis data includes
cropping a region occupied by teeth in the first image to extract the region as a second image;
analyzing whether there is a symptom of at least one of cavities, tartar, tooth fracture, tooth discoloration, residual baby teeth, and tooth loss based on the second image and diagnosing the presence or absence of the oral disease in the tooth part to generate the actual diagnosis data;
cropping a region occupied by gums in the first image to extract the area as a third image; and
analyzing whether there is a symptom of at least one of gum inflammation and gum tumor based on the third image, and diagnosing the presence or absence of the oral disease in the gum part to generate the actual diagnosis data.

6. The method of claim 1, wherein the generating of the basic result data includes analyzing the analysis image for each part with the naked eye based on basic information of the examination subject included in the basic condition information, and classifying the oral disease into presence and labeling the basic diagnosis data when there is tartar on teeth of the examination subject or inflammation in gums of the examination subject.

7. A system for diagnosing an oral disease of a companion animal, comprising:
a user terminal configured to generate condition measurement information including actual photographing information acquired from an examination subject; and
a health management server configured to repeatedly learn health standard data generated by matching basic condition information with basic result data and analyze the condition measurement information to generate health result data for the examination subject,
wherein the health management server
extracts an actual analysis image from the actual photographing information, analyzes an actual analysis image for each part extracted from the actual analysis image simultaneously to diagnose the presence or absence of the oral disease for the examination subject and generate actual diagnosis data, generates the health result data including actual determination data generated by determining a disease-specific progression stage of the oral disease based on the actual diagnosis data, preprocesses an image included in basic photographing information included in the basic condition information, extracts an analyzable analysis image from the image, extracts an analysis image for each part for analyzing a plurality of diseases from the analysis image and diagnosing the presence or absence of the oral disease for the analysis image for each part to generate basic diagnosis data, determines a disease-specific progression stage for the analysis image for each part based on the basic diagnosis data to generate basic determination data, and generates the basic result data including the basic diagnosis data and the basic determination data corresponding to the basic diagnosis data.

8. A computer program stored in a computer-readable recording medium coupled to a computer as hardware to perform the method of any one of claims 1 to 6.
